Europäisches Patentamt

European Patent Office

Office européen des brevets

(19)

(11) Veröffentlichungsnummer : **0 123 841**
**B1**

(12) **EUROPÄISCHE PATENTSCHRIFT**

(45) Veröffentlichungstag der Patentschrift :
**20.09.89**

(51) Int. Cl.⁴ : **A 61 K 31/505**

(21) Anmeldenummer : **84102771.7**

(22) Anmeldetag : **14.03.84**

(54) **Arzneimittel mit einer die atheroprotektiven High Density Lipoproteine steigernden Wirkung, enthaltend 2,6-Bis-diäthanolamino-4,8-dipiperidino-pyrimido (5,4-d)-pyrimidin.**

(30) Priorität : **29.03.83 DE 3311355**

(43) Veröffentlichungstag der Anmeldung :
**07.11.84 Patentblatt 84/45**

(45) Bekanntmachung des Hinweises auf die Patenterteilung : **20.09.89 Patentblatt 89/38**

(84) Benannte Vertragsstaaten :
**BE CH DE FR GB IT LI LU NL SE**

(56) Entgegenhaltungen :
**ROTE LISTE, BUNDESVERBAND DER PHARMAZEU-
TISCHEN INDUSTRIE, 1982, Seite 119, Cantor, Aulendorf, DE;
Moderne Arzneimittel (3Auflage) Wissentschaftliche
Verlagsgesellschaft mbH Stuttgart (1980)
Progress in cardiovascular diseases vol. XXV11
(1984) pp1-20
Circulation 69 (1984) pp 325-337
European Journal of clinical investigation 8 (1979) pp
107-109
Clin. Chem. 27 (1981) pp 653-662
Atherosclerosis (1979) pp 315-327
7th int. Symposium on Atherosclerosis, Melbourne
Ref. 428
Arteriosclerosis vol. 6 (1986) pp 558a
Prospekt "Persantin, Persantin forte" zur Behandlung der koronaren Herzkrankheit und Verhütung
der Thrombose
The New England Journal of Medicine 20(1988)pp
1279-1281**

(73) Patentinhaber : **Dr. Karl Thomae GmbH
Postfach 1755
D-7950 Biberach (Riss) (DE)**

(72) Erfinder : **Herce Munoz, Millán
"La Rosaleda" Torre Centro
1o B. Sevilla (6) (ES)**
Erfinder : **Machuca Jiménez, Guillermo
Asuncion, 19
3o A. Sevilla (11) (ES)**
Erfinder : **Mora Rocha, Ildefonso
Avda. Republica Argentina, 7
5o D. Sevilla (11) (ES)**
Erfinder : **Fernández Garcia, Juan-José
Avda. J.M. Martinez Sánchez Arjona, 18
2o A. Sevilla (10) (ES)**
Erfinder : **Rodriguez Alvarez, José
Asuncion, 73
2o D. Sevilla (11) (ES)**

**Beschreibung**

Es ist bekannt, daß die Verbindung 2,6-Bis-diäthanolamino-4,8-dipiperidino-pyrimido [5,4-d] pyrimidin (generic name : Dipyridamol) unter dem Handelsnamen Persantin in der Therapie zur Behandlung der koronaren Herzkrankheit und zur Verhütung der Thrombose eingesetzt wird, insbesondere jedoch bei der Reinfarktprophylaxe (siehe Circulation Vol 62 III, 449-461 (1980)). Bei diesen Indikationen wird eine Einzeldosis von 25 bis 75 mg gegebenenfalls bis zu 8 x täglich empfohlen. Außerdem haben mehrere Kombinationen mit dieser Verbindung, z. B. mit Acetylsalicylsäure, Clofibrat, Digoxin, Toliprolol-hydrochlorid, Phenobarbital und Oxazepam, in der Therapie Eingang gefunden, welche pro Einzeldosis ebenfalls 25 bis 75 mg 2,6-Bis-diäthanolamino-4,8-dipiperidino-pyrimido [4,5-d] pyrimidin enthalten. Desweiteren ist aus der Literatur bekannt, daß Dipyridamol auch eine senkende Wirkung auf den Gesamtcholesterinspiegel aufweist (siehe Ärztliche Forschung XIII, 196-200 (1959)).

Überraschenderweise wurde nun gefunden, daß 2,6-Bis-diäthanolamino-4,8-dipiperidino-pyrimido [5,4-d] pyrimidin und dessen physiologisch verträgliche Säureadditionssalze mit anorganischen oder organischen Säuren, zweckmäßigerweise jedoch sein Hydrochlorid, auch das Lipoproteinprofil günstig beeinflussen. Das heißt, die atheroprotektiven High Density Lipoproteine (HDL) werden erhöht und die atherogenen β-Lipoproteine (VLDL und LDL) gesenkt. Dies führt zu einer Senkung des Quotienten LDL/HDL, des sogenannten atherogenen Index.

Dies ist umso überraschender, da von den zur gleichen Indikation eingesetzten β-Blockern bekannt ist, daß sie das Verhältnis der atherogenen Low Density Lipoproteine (LDL) zu den atherogenen High Density Lipoproteinen (HDL) ungünstig beeinflussen, das heißt HDL erniedrigen und Very Low Density Lipoproteine (VLDL) und Serumtriglyceride erhöhen, womit das atherogene Risiko erhöht wird (siehe Brit. Med. Journal 284, 1145-1148 (1982)).

Gegenstand der vorliegenden Anmeldung ist somit die Verwendung von 2,6-Bis-diäthanolamino-4,8-dipiperidinopyrimido [5,4-d]-pyrimidin oder von dessen physiologisch verträglichen Säureadditionssalzen mit einer anorganischen oder organischen Säure zur Herstellung eines Arzneimittels mit einer steigernden atheroprotektiven Wirkung auf die High Density Lipoproteine.

Die auf diese Weise hergestellten Arzneimittel sind daher insbesondere zur Behandlung und Prophylaxe der Atherosclerose geeignet.

Die Verwendung der Arzneimittel wurde klinisch wie folgt geprüft :

57 Patíenten (27 Frauen + 30 Männer) im Alter zwischen 40 und 80 Jahren (Mittelwert : 61,4 Jahren) erhielten 3 x täglich je 100 mg Dipyridamol (jeweils zu den drei Hauptmahlzeiten) 5 Monate lang. Bei 17 Patienten mußte die Tagesdosis auf 250 mg reduziert werden, da aufgrund der gefäßerweiternden Wirkung sekundäre Kopfschmerzen auftraten bzw. aufgrund einer speziellen Sensibilität des Patienten. Die Bestimmung der Lipoproteinfraktionen (VLDL und LDL) und HDL erfolgte mit der Phosphorwolfram-$Mg^{++}$ Methode.

Bei der Auswertung der Befunde wurden die Lipoproteinwerte eines jeden Patienten vor Beginn der Behandlung mit Dipyridamol und nach einer 2- bzw. 4-monatigen Behandlung bestimmt. Zur Bewertung der Meßergebnisse wurde eine Varianzanalyse für verbundene Stichproben verwendet.

Die nachfolgenden Tabellen enthalten die gefundenen Werte :

HDL-Fraktion :

| | Mittelwert $\bar{x}$ | Differenz | |
|---|---|---|---|
| | | absolut | in % |
| vor Behandlung | 42,1 | - | - |
| nach 2 Monaten | 46,9 | + 4,8 | + 11,0 |
| nach 4 Monaten | 50,0 | + 7,9 | + 19,0 |

VLDL + LDL-Fraktionen :

| | Mittelwert X | Differenz | |
|---|---|---|---|
| | | absolut | in % |
| vor Behandlung | 230,9 | - | - |
| nach 2 Monaten | 198,9 | - 32,0 | - 13,9 |
| nach 4 Monaten | 194,8 | - 36,1 | - 15,6 |

Die in den Tabellen aufgeführten Unterschiede sind statistisch signifikant (p < 5 %).

Aus den obigen Tabellen geht hervor, daß während der Behandlung mit Dipyridamol die atheroprotektiven HDL um bis zu 19 % erhöht wurden, während die atherogenen β-Lipoproteine (VLDL und LDL) um bis zu 15,6 % gesenkt wurden.

Ferner ist bekannt, daß Dipyridamol praktisch untoxisch ist. So zeigten in chronischen Toxizitätsuntersuchungen Ratten nach dreimonatiger täglicher Verfütterung von 100 mg/kg und Hunde nach 20 mg/kg täglich über sechs Monate weder im Verhalten noch im Sektionsbild krankhafte Veränderungen.

Die zur Erzielung der erfindungsgemäßen Wirkung erforderliche Dosis beträgt am Erwachsenen zweckmäßigerweise 3- bis 4 mal täglich 50 bis 150 mg, vorzugsweise jedoch 80 bis 100 mg, die Tagesdosis liegt also zweckmäßigerweise zwischen 150 und 600 mg, vorzugsweise jedoch zwischen 225 und 400 mg. Hierzu läßt sich die Wirksubstanz zusammen mit einem oder mit mehreren inerten üblichen Trägerstoffen in die üblichen galenischen Zubereitungen und deren Depot-Formen wie Tabletten, Dragées, Kapseln oder Pulvern einarbeiten.

Die nachfolgenden Beispiele sollen die Erfindung näher erläutern :

## Beispiel 1

Dragées mit 75 mg 2,6-Bis-diäthanolamino-4,8-dipiperidino-pyrimido[5,4-d]pyrimidin

| | | |
|---|---|---|
| Wirksubstanz | (1) | 7,5 kg |
| Lactose | (2) | 4,2 kg |
| Maisstärke | (3) | 2,4 kg |
| Polyvinylpyrrolidon | (4) | 0,7 kg |
| Magnesiumstearat | (5) | 0,2 kg |
| | | 15,0 kg |

Herstellung

Die Stoffe (1), (2), (3) und (4) werden trocken gemischt und danach mit Wasser befeuchtet. Das Granulat gibt man durch ein Sieb mit 1,2 mm-Maschenweite und trocknet es bei 45 °C. Nach erneuter Siebung durch dasselbe Sieb fügt man (5) hinzu, mischt und verpreßt zu Dragéekernen von 8,0 mm Durchmesser und 150 mg Gewicht.

Die Kerne werden mit 5 kg Dragiersuspension, die aus Zucker, Talk, Gummi arabicum und Titandioxid besteht, auf 200 mg Gewicht dragiert. Zum Schluß poliert man die Dragées mit Polyäthylenglykol 6000 und Carnaubawachs.

## Beispiel 2

Filmtabletten mit 80 mg 2,6-Bis-diäthanolamino-4,8-dipiperidino-pyrimido[5,4-d]pyrimidin

| | |
|---|---|
| Wirksubstanz | 8,0 kg |
| Weinsäure | 6,0 kg |
| Mikrokristalline Cellulose | 4,5 kg |
| Maisstärke | 0,7 kg |
| Magnesiumstearat | 0,3 kg |
| | 19,5 kg |

Herstellung

Wirksubstanz wird mit Weinsäure vermischt und feucht granuliert. Dem getrockneten Granulat werden die restlichen Hilfsstoffe zugemischt. Man verpreßt das Granulat zu runden, bikonvexen Kernen von 8 mm Durchmesser. Die Kerne werden zur Geschmacksabdeckung mit einem Hydroxypropylmethylcellulose-Überzug im Dragierkessel versehen.

Beispiel 3

Depot-Kapseln mit 100 mg 2,6-Bis-diäthanolamino-4,8-dipiperidino-pyrimido [5,4-d] pyrimidin

In einem Wirbelschichtgranuliergerät werden 15 kg Wirksubstanz mit 11 kg Bernsteinsäurepulver gemischt. Mit Hilfe von 30 kg einer 5 %igen Hydroxypropylmethylcellulose-Lösung wird bei langsamen Sprühen ein kugelartiges Aufbaugranulat hergestellt. Den Siebanteil zwischen 0,6 und 1,0 mm überzieht man in einem Dragierkessel mit einer isopropanolischen Lösung von Methacrylsäure-Methacrylsäureester-Mischpolymerisat solange, bis 7 % Retardlack (bezogen auf das Granulat) aufgesprüht sind. Nach Bestimmung des Gehaltes wird die 100 mg Dipyridamol entsprechende Menge Granulat in Hartgelatinekapseln abgefüllt.

Beispiel 4

Depot-Kapseln mit 100 mg 2,6-Bis-diäthanolamino-4,8-dipiperidino-pyrimido [5,4-d] pyrimidin

In einem Dragierkessel wird auf 20 kg Weinsäurekristalle einer Teilchengröße von 0,7 bis 0,8 mm jeweils nach Befeuchten mit einer Polyvinylpyrrolidonlösung in kleinen Anteilen eine Pulvermischung aus Wirksubstanz und Talkum aufgetragen. Wenn die entstehenden Wirkstoffpellets einen Wirksubstanz-Gehalt von 46 % aufweisen, werden sie in einem rasch rotierenden Kessel mit einem Lackgemisch aus Methacrylsäure-Methacrylsäureester Copolymer und Hydroxypropylmethylcellulosephthalat, gelöst in Aceton und Isopropanol, besprüht. Die auf die Pellets bezogene Lackmenge beträgt 9 %. Nach Bestimmung des Gehalts wird die 100 mg Dipyridamol entsprechende Menge Pellets in Hartgelatinekapseln abgefüllt.

**Patentansprüche**

1. Verwendung von 2,6-Bis-diäthanolamino-4,8-dipiperidino-pyrimido-[5,4-d] pyrimidin oder dessen physiologisch verträgliches Säureadditionssalz zur Herstellung eines Arzneimittels mit einer atheroprotektiven Wirkung.

2. Verwendung von 2,6-Bis-diäthanolamino-4,8-dipiperidino-pyrimido-[5,4-d] pyrimidin oder dessen physiologisch verträgliches Säureadditionssalz zur Herstellung eines Arzneimittels mit einer die atheroprotektiven High Density Lipoproteine steigernden Wirkung.

**Claims**

1. Use of 2,6-bis-diethanolamino-4,8-dipiperidino-pyrimido-[5,4-d] pyrimidine or a physiologically acceptable acid addition salt thereof for producing pharmaceutical compositions having an atheroprotective effect.

2. Use of 2,6-bis-diethanolamino-4,8-dipiperidino-pyrimido-[5,4-d] pyrimidine or a physiological acceptable acid addition salt thereof with an inorganic or organic acid for producing a pharmaceutical composition with the effect of increasing atheroprotective high density lipoproteins.

**Revendications**

1. Utilisation de la 2,6-bis-diéthanolamino-4,8-dipipéridino-pyrimido [5,4-d] pyrimidine ou de son sel d'addition d'acide physiologiquement supportable pour la fabrication d'un médicament présentant une activité athéroprotectrice.

2. Utilisation de la 2,6-bis-diéthanolamino-4,8-dipipéridino-pyrimido [5,4-d] pyrimidine ou de son sel d'addition d'acide physiologiquement supportable pour la fabrication d'un médicament ayant une activité augmentant les lipoprotéines haute densité athéroprotectrices ou à activité athéroprotectrice croissante des lipoprotéines haute densité.